# EUROPÄISCHE PATENTANMELDUNG

(11) **EP 1 582 601 A1**
(43) Veröffentlichungstag der Anmeldung: **05.10.2005**
(21) Anmeldenummer: 04450078.3
(22) Anmeldetag: 01.04.2004
(51) Int. Cl.: C22B 9/02, C22B 26/22, F27B 14/02, F27D 23/00, G01N 1/12

(54) **Schmelzvorrichtung zur Bestimmung von nichtmetallischen Einschlüssen in metallischem Material**

(71) Anmelder: TCG UNITECH Aktiengesellschaft, 4560 Kirchdorf/Krems (AT)
(72) Erfinder: Widegger, Reinhard, Dipl.-Ing., 4531 Kematen/Krems (AT)
(74) Vertreter: Babeluk, Michael

(57) **Zusammenfassung**

Die Erfindung betrifft ein Verfahren zur Bestimmung von nichtmetallischen Einschlüssen in metallischen Ausgangsmaterialien (17) wie Halbzeuge, Barren, Pressbolzen oder Masseln, insbesondere aus Magnesiumlegierungen, mit folgenden Schritten:
- Einbringen des zu untersuchenden Ausgangsmaterials (17) in einen Schmelztiegel (3) einer Tiegeleinheit (2);
- Verschließen der Tiegeleinheit (2) durch Aufsetzen eines Erstarrungstiegels (4) auf den Schmelztiegel (3), wobei zwischen Schmelztiegel (3) und Erstarrungstiegel (4) eine Filterhalterung (9) mit einem Filter (10) angeordnet wird;
- Einsetzen der Tiegeleinheit (2) in einem Tiegelofen (12);
- Erwärmen des Schmelztiegels (3) der Tiegeleinheit (2) mit dem Tiegelofen (12) bis das Ausgangsmaterial (17) aufgeschmolzen ist;
- Schwenken des Tiegelofens (12) samt Tiegeleinheit (2) um etwa 180° um eine etwa horizontale Achse (18), so dass das aufgeschmolzene Material unter Passieren des Filters (10) vom Schmelztiegel (3) in den Erstarrungstiegel (4) fließt;
- Abkühlen des Erstarrungstiegels (4);
- Entnehmen der Tiegeleinheit (2) aus dem Tiegelofen (12) und Zerlegen der Tiegeleinheit (12);
- Untersuchen des Filtrats und/oder des gefilterten Materials.

Dadurch lassen sich auf einfache Weise nichtmetallische Einschlüsse in Metallen bestimmen.

## Beschreibung

Die Erfindung betrifft ein Verfahren sowie eine Vorrichtung zur Bestimmung von nichtmetallischen Einschlüssen in metallischen Ausgangsmaterialien wie Halbzeuge, Barren, Pressbolzen oder Masseln, insbesondere aus Magnesiumlegierungen.

Magnesium wird zunehmend als Werkstoff, insbesondere zur Herstellung von Gussteilen, eingesetzt. Magnesium wird, ähnlich wie Aluminium, aus einem Elektrolyseverfahren gewonnen und zu Barren, Pressbolzen oder Masseln vergossen. Diese werden vor Ihrer Weiterverarbeitung in speziellen Schmelzöfen eingeschmolzen. Die Qualität des Magnesiums unterliegt dabei herkunftsabhängig starker Schwankung.

Aus der EP 0 738 334 B1 ist eine Vorrichtung, sowie ein Verfahren zum Aufbereiten bzw. Reinigen von Magnesiumschmelze bekannt. Die Magnesiumschmelze wird dabei in mehrere hintereinander angeordnete Kammern geleitet, wobei die Magnesiumschmelze einen Keramikfilter zur Reinigung passiert.

Die US 5,914,440 A beschreibt ein Verfahren und eine Vorrichtung zum Entfernen von Einschlüssen in einer Magnesiumschmelze, wobei ein Filter eingesetzt wird.

Auch die EP 0 642 594 B1 offenbart ein Verfahren und eine Vorrichtung zur Entfernung von Partikeln in einem geschmolzenen Metall mittels einer Filtereinrichtung.

Ein Keramikfilter zum Filtern von geschmolzenen Metallen wird in der US 5,004,545 A beschrieben.

Die bekannten Verfahren zum Aufbereiten und Reinigen der Magnesiumschmelze sind relativ aufwendig und eignen sich nicht zur Eingangskontrolle von Magnesiumrohmaterial.

Aufgabe der Erfindung ist es, ein Verfahren sowie eine Vorrichtung zur Durchführung von Kontrollen an metallischen Stoffen durchzuführen.

Erfindungsgemäß wird dies durch ein Verfahren mit folgenden Schritten erreicht:
- Einbringen des zu untersuchenden Ausgangsmaterials in einen Schmelztiegel einer Tiegeleinheit;
- Verschließen der Tiegeleinheit durch Aufsetzen eines Erstarrungstiegels auf den Schmelztiegel, wobei zwischen Schmelztiegel und Erstarrungstiegel eine Filterhalterung mit einem Filter angeordnet wird;
- Einsetzen der Tiegeleinheit in einem Tiegelofen;
- Erwärmen des Schmelztiegels der Tiegeleinheit mittels dem Tiegelofen bis das Ausgangsmaterial aufgeschmolzen ist;
- Schwenken des Tiegelofens samt Tiegeleinheit um etwa 180° um eine etwa horizontale Achse, so dass das aufgeschmolzene Material unter Passieren des Filters vom Schmelztiegel in den Erstarrungstiegel fließt;
- Abkühlen des Erstarrungstiegels;
- Entnehmen der Tiegeleinheit aus dem Tiegelofen und Zerlegen der Tiegeleinheit;
- Untersuchen des Filtrats und/oder des gefilterten Materials.

Die Durchführung des Verfahrens erfolgt mit einem Tiegelofen und einer aus Schmelz- und Erstarrungstiegel bestehenden geschlossenen Tiegeleinheit, wobei innerhalb der Tiegeleinheit zwischen Schmelz- und Erstarrungstiegel ein Filterhalter für einen einsetzbaren Filter angeordnet ist, wobei vorzugsweise die Filterhalterung als konischer Zwischenring zwischen Schmelz- und Erstarrungstiegel ausgebildet ist. Vor oder während des Aufheizvorganges wird über eine Gasleitung der Innenraum der Tiegeleinheit evakuiert und mit einem Inertgas, beispielsweise Argon, gespült und danach unter leichten Überdruck gesetzt, um eine Oxidation des Materials im Tiegel zu verhindern, was das Ergebnis verfälschen würde. Nach Aufschmelzen des Materials, was abhängig von der Materialart und -menge bis zu 1,5 Stunden dauern kann, wird die Vorrichtung um 180° um seine Achse gedreht, und das Material läuft über den Filterhalter mit dem Filter in den Erstarrungstiegel.

Das Gasrohr zur Evakuierung und zur Zuführung des Inertgases ist vorzugsweise knapp unter dem Randflansch des Erstarrungstiegels angebracht, wodurch gemeinsam mit dem konisch geformten Zwischenring und bei geeigneter Drehrichtung der Vorrichtung ein Kontakt der Druckleitung mit der Schmelze vermieden werden kann.

Anschließend wird die Heizung des Tiegelofens abgeschaltet und Pressluft durch den Innenraum des Tiegelofens geleitet. Je nach Menge und Art des Materials dauert die Kühlphase etwa 45 bis 60 Minuten.

Nach Abkühlung des Erstarrungstiegels auf etwa 350° kann die Tiegeleinheit dem Tiegelofen entnommen und zur Untersuchung des Filtrats geöffnet werden.

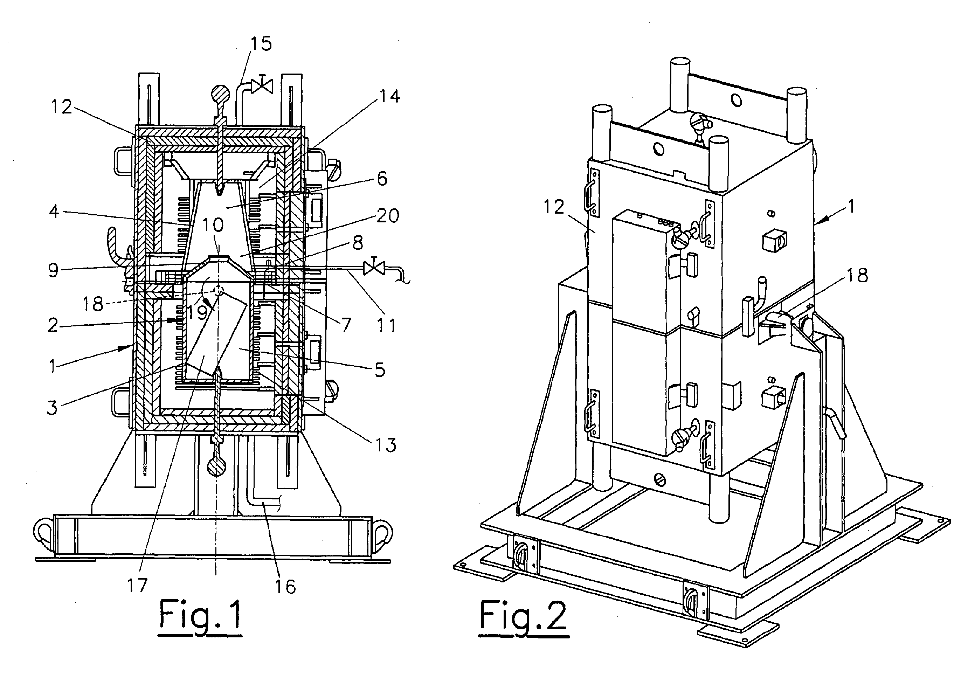

Die Erfindung wird im Folgen anhand der Figur näher erläutert. Es zeigen
- Fig. 1: eine erfindungsgemäße Vorrichtung in einem Längsschnitt, und
- Fig. 2: die Vorrichtung in einer Schrägansicht.

Die Figur zeigt schematisch eine Vorrichtung 1 zur Bestimmung von nichtmetallischen Einschlüssen in metallischen Ausgangsmaterialien 17, wie Halbzeuge, Masseln, Barren, Pressbolzen oder dergleichen, insbesondere in Magnesiumverbindungen.

Die Vorrichtung 1 besteht aus einer Tiegeleinheit 2 mit einem Schmelztiegel 3 und einem Erstarrungstiegel 4, welche einen Hohlraum 20 mit einer Schmelzkammer 5 und einer Erstarrungskammer 6 einschließen. Schmelztiegel 3 und Erstarrungstiegel 4 sind über Randflansche 7, 8 miteinander verbunden. Im Bereich der Randflansche 7, 8 ist zwischen Schmelztiegel 3 und Erstarrungstiegel 4 ein Filterhalter 9 mit einem Filter 10 aus Keramikmaterial eingesetzt, wobei Schmelzkammer 5 und Erstarrungskammer 6 durch den Filterhalter 9 und den Filter 10 voneinander getrennt sind. Der Filterhalter 9 ist im Ausführungsbeispiel durch einen konischen Zwischenring zwischen Schmelztiegel 3 und Erstarrungstiegel 4 gebildet.

In die Erstarrungskammer 6 mündet nahe dem Randflansch 8 eine Gasleitung 11 ein.

Die aus Schmelztiegel 3 und Erstarrungstiegel 4 gebildete Tiegeleinheit 2 ist während des Schmelz- und Erstarrungsvorganges in einen Tiegelofen 12 eingesetzt, welcher zumindest im Bereich des Schmelztiegels 3 durch Heizelemente 13 beheizbar ist. Der Innenraum 14 ist mit Kühlleitungen 15, 16 verbunden.

Das zu untersuchende Ausgangsmaterial 17 wird in den Schmelztiegel 3 der Vorrichtung 1 eingebracht und in den Filterhalter 9 ein passender Filter 10, beispielsweise aus Keramik, eingesetzt, der die nichtmetallischen Einschlüsse, welche vornehmlich Oberflächenoxide sind, abfiltern kann. Der Erstarrungstiegel 4 wird auf den Schmelztiegel 3 verkehrt aufgesetzt, so dass durch Schmelztiegel 3 und Erstarrungstiegel 4 eine Schmelzkammer 5 und eine Erstarrungskammer 6 definiert werden. Die Tiegeleinheit 2 wird sodann in einen Tiegelofen 12 eingebaut und der Tiegelofen 12 auf eine Temperatur von etwa 700°C bis 725°C erwärmt. Dabei wird zu Beginn des Aufheizvorganges mittels der Gasleitung 11 Luft aus der Tiegeleinheit 2 mittels einer nicht weiter dargestellten Vakuumpumpe herausgepumpt und der Hohlraum 20 der Tiegeleinheit 2 mit einem nicht reaktiven Gas, beispielsweise Argon, gespült. Der Hohlraum 20 der Tiegeleinheit 2 wird anschließend unter leichtem Überdruck bezüglich der Umgebung, beispielsweise auf 1,5 bar absolut, gesetzt. Dadurch wird eine Oxidation des Materials innerhalb der Tiegeleinheit 2 verhindert, welche das Ergebnis verfälschen könnte.

Nach Aufschmelzen des Ausgangsmaterials 17, was abhängig von Materialart und -menge bis zu 1,5 Stunden dauern kann, wird die Vorrichtung 1 um 180° um eine horizontale Achse 18 gedreht, wodurch das geschmolzene Material über den konischen Filterhalter 9 und den Filter 10 in den Erstarrungstiegel 6 fließt. Da das Gasrohr 11 nahe dem Flansch 8 in den Erstarrungstiegel 6 mündet, wird bei passender Drehrichtung 19 zusammen mit dem konisch ausgebildeten Filterhalter 9 eine Kontaktierung der Gasleitung 11 mit der Schmelze vermieden.

Danach wird die Heizeinrichtung 13 des Tiegelofens 12 abgeschaltet und über die Kühlleitung 15 Pressluft in den Innenraum 14 des Tiegelofens 12 geleitet, um die Tiegeleinheit 2 abzukühlen. Die Kühlluft kann über die Kühlleitung 16 abgeleitet werden. Je nach Menge und Art des Ausgangsmaterials 17 dauert die Kühlphase 45 bis 60 Minuten.

Sobald etwa 350°C im Erstarrungstiegel 6 erreicht sind, kann die Tiegeleinheit 2 dem Tiegelofen 12 entnommen und zur Analyse des Filtrates geöffnet werden.

Durch die relativ langsame Kühlung ist die Korngröße im gefilterten Material im Erstarrungstiegel 6 mehr oder weniger deutlich sichtbar. Auf jeden Fall sollte die Oberfläche des gefilterten Materials glänzend sein, da sonst eine unbemerkte Oxidation durch Luftzufuhr und damit eine Ergebnisverfälschung geschehen ist.

Im Filterhalter 9 und dem Filter 10 bildet sich eine Kruste aus Oxiden, welche durch den Filter 10 zurückgehalten wurden. Für die Beurteilung der Güte des Ausgangsmaterials 17 wird diese Kruste herangezogen. Dabei werden Farbe und Glanz, Geruch, Aussehen, Oberflächenbeschaffenheit, Menge und Form untersucht.

Ein nicht vollständiges Durchlaufen der Schmelze durch den Filter 10 deutet auf flächige Oxide und Schweboxide hin, welche eine schlechte Materialqualität indizieren. Ist die Schmelze vollständig durch den Filter 10 gelaufen und der Film am Filterhalter 9 dünn und glänzend, weist dies auf eine gute Qualität des Ausgangsmaterials 17 hin.

## Patentansprüche

1. Vorrichtung (1) zur Bestimmung von nichtmetallischen Einschlüssen in metallischen Ausgangsmaterialien (17) wie Halbzeuge, Barren, Pressbolzen oder Masseln, insbesondere aus Magnesiumlegierungen, mit einem Tiegelofen (12) und einer in den Tiegelofen (12) einsetzbaren, aus einem Schmelz- und einem Erstarrungstiegel (3, 4) bestehenden Tiegeleinheit (2), wobei Schmelz- und Erstarrungstiegel (3, 4) entlang ihrer einander zugekehrten Randflansche (7, 8) miteinander lösbar verbunden sind, und eine Schmelz- und eine Erstarrungskammer (5, 6) definieren, welche durch eine vorzugsweise zwischen Schmelz- und Erstarrungstiegel (3, 4) angeordnete Filterhalterung (9) zur Aufnahme eines Filters (10) getrennt sind.

2. Vorrichtung (1) nach Anspruch 1, **dadurch gekennzeichnet, dass** die Filterhalterung (9) als konischer Zwischenring zwischen Schmelz- und Erstarrungstiegel (3, 4) ausgebildet ist.

3. Vorrichtung (1) nach Anspruch 1 oder 2, **dadurch gekennzeichnet, dass** der Innenraum (14) des Tiegelofens (12) und/oder die Schmelz- und Erstarrungskammern (5, 6) durch eine Gasleitung (11) evakuierbar und/oder mit Inertgas befüllbar ist.

4. Vorrichtung (1) nach Anspruch 3, **dadurch gekennzeichnet, dass** die Gasleitung (11) in den Erstarrungstiegel (4) einmündet, wobei vorzugsweise die Einmündung im Bereich eines Randflansches (8) des Erstarrungstiegels (4) angeordnet ist.

5. Vorrichtung (1) nach einem der Ansprüche 1 bis 4, **dadurch gekennzeichnet, dass** der Filter (10) aus einem keramischen Material besteht.

6. Vorrichtung (1) nach einem der Ansprüche 1 bis 5, **dadurch gekennzeichnet, dass** die Tiegeleinheit (2) um zumindest 180° um eine im Wesentlichen horizontale Achse (18) schwenkbar sind.

7. Vorrichtung (1) nach Anspruch 6, **dadurch gekennzeichnet, dass** der Tiegelofen (12) samt Tiegeleinheit (2) um zumindest 180° um die im Wesentlichen horizontale Achse (18) schwenkbar angeordnet ist.

8. Vorrichtung (1) nach einem der Ansprüche 1 bis 7, **dadurch gekennzeichnet, dass** der Innenraum (14) des Tiegelofens (12) mit Kühlluft spülbar ist, wobei zumindest eine Kühlleitung (15, 16) in den Innenraum (14) einmündet.

9. Verfahren zur Bestimmung von nichtmetallischen Einschlüssen in einem metallischen Ausgangsmaterialien (17) wie Halbzeuge, Barren, Pressbolzen oder Masseln, insbesondere aus Magnesiumlegierungen, mit folgenden Schritten:
- Einbringen des zu untersuchenden Ausgangsmaterial (17) in einen Schmelztiegel (3) einer Tiegeleinheit (2);
- Verschließen der Tiegeleinheit (2) durch Aufsetzen eines Erstarrungstiegels (4) auf den Schmelztiegel (3), wobei zwischen Schmelztiegel (3) und Erstarrungstiegel (4) eine Filterhalterung (9) mit einem Filter (10) angeordnet wird;
- Einsetzen der Tiegeleinheit (2) in einem Tiegelofen (12);
- Erwärmen des Schmelztiegels (3) der Tiegeleinheit (2) mit dem Tiegelofen (12) bis der Ausgangsmaterial (17) aufgeschmolzen ist;
- Schwenken des Tiegelofens (12) samt Tiegeleinheit (2) um etwa 180° um eine etwa horizontale Achse (18), so dass das aufgeschmolzene Material unter Passieren des Filters (10) vom Schmelztiegel (3) in den Erstarrungstiegel (4) fließt;
- Abkühlen des Erstarrungstiegels (4);
- Entnehmen der Tiegeleinheit (2) aus dem Tiegelofen (12) und Zerlegen der Tiegeleinheit (12);
- Untersuchen des Filtrats und/oder des gefilterten Materials.

10. Verfahren nach Anspruch 9, **dadurch gekennzeichnet, dass** der Tiegelofen (12) auf eine Temperatur von etwa 700°C bis 725°C erwärmt wird.

11. Verfahren nach Anspruch 9 oder 10, **dadurch gekennzeichnet, dass** vor oder während des Aufheizvorganges die Tiegeleinheit (2) evakuiert, mit Inertgas gespült und gefüllt wird, wobei vorzugsweise ein Überdruck im Innenraum (14) gegenüber der Umgebung eingestellt wird.

12. Verfahren nach einem der Ansprüche 9 bis 11, **dadurch gekennzeichnet, dass** der Innenraum (14) des Tiegelofens (12) während der Abkühlphase mit Kühlluft gespült wird.
